(19) Europäisches Patentamt
European Patent Office
Office européen des brevets

(11) Veröffentlichungsnummer: **0 396 999 B1**

# (12) EUROPÄISCHE PATENTSCHRIFT

(45) Veröffentlichungstag der Patentschrift: **13.04.94**

(51) Int. Cl.[5]: **C07C 229/22**, C11D 3/33

(21) Anmeldenummer: **90108201.6**

(22) Anmeldetag: **30.04.90**

Die Akte enthält technische Angaben, die nach dem Eingang der Anmeldung eingereicht wurden und die nicht in dieser Patentschrift enthalten sind.

## (54) 2-Methyl- und 2-Hydroxymethyl-serin-N,N-diessigsäure und ihre Derivate.

(30) Priorität: **06.05.89 DE 3914980**

(43) Veröffentlichungstag der Anmeldung:
**14.11.90 Patentblatt 90/46**

(45) Bekanntmachung des Hinweises auf die Patenterteilung:
**13.04.94 Patentblatt 94/15**

(84) Benannte Vertragsstaaten:
**DE FR GB IT**

(56) Entgegenhaltungen:
**EP-A- 0 287 846**

**ACTA CHIM. HUNG., Tomus 21, 1959, L. ERDEY "Herstellung einiger neuer komplexbildender Verbindungen und Bestimmung ihrer Konstanten", S. 327-332**

(73) Patentinhaber: **BASF Aktiengesellschaft**
**Carl-Bosch-Strasse 38**
**D-67063 Ludwigshafen(DE)**

(72) Erfinder: **Oftring, Alfred, Dr.**
**Im Roehring 49**
**D-6702 Bad Duerkheim(DE)**
Erfinder: **Birnbach, Stefan, Dr.**
**Budapester Strasse 45**
**D-6700 Ludwigshafen(DE)**
Erfinder: **Baur, Richard, Dr.**
**Nelkenstrasse 1**
**D-6704 Mutterstadt(DE)**
Erfinder: **Gousetis, Charalampos, Dr.**
**Carl-Bosch-Strasse 98**
**D-6700 Ludwigshafen(DE)**
Erfinder: **Trieselt, Wolfgang, Dr.**
**Alwin-Mittasch-Platz 1**
**D-6700 Ludwigshafen(DE)**

EP 0 396 999 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 2-Methyl- und 2-Hydroxymethyl-serin-N,N-diessigsäure und ihre Derivate der allgemeinen Formel I

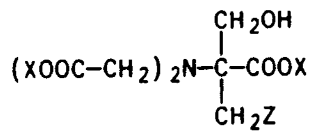

I

in der X für Wasserstoff, Alkalimetalle oder Ammonium, das durch $C_1$-$C_4$-Alkylgruppen oder $C_1$-$C_4$-Hydroxyalkylgruppen substituiert sein kann, steht und Z Wasserstoff oder eine Hydroxylgruppe bezeichnet. Außerdem betrifft die Erfindung ein Verfahren zur Herstellung der Verbindungen I sowie ihre Verwendung als Komplexbildner für Schwermetall- oder Erdalkalimetallionen und als Bleichmittelstabilisatoren oder Gerüststoffe in Wasch- und Reinigungsmitteln und die die Verbindungen I enthaltenden hierzu entsprechenden Mittel.

Die allgemeine Lehre der EP-A 001 310 betrifft Waschmittelzubereitungen aus anionischen, nichtionischen, amphoteren oder zwitterionischen Detergentien, Polyphosphonaten und Carboxylgruppen enthaltenden Verbindungen, wobei die für die letztgenannten Verbindungen angegebene allgemeine Formel auch die Serinderivate I umfaßt, ohne daß jedoch Näheres hierüber offenbart wird.

Erdey beschreibt in Acta Chim. Hung., Tomus 21, 1959, S. 327-332, die komplexbildenden Eigenschaften von D,L-Serin-N,N-diessigsäure. Dabei wurden für die dargestellten Komplexe mit Erdalkalimetallionen geringere Stabilitätswerte als beispielsweise für die entsprechenden Komplexe mit Nitrilotriessigsäure festgestellt.

Aufgabe der vorliegenden Erfindung war es deshalb, neue Komplexbildner für Schwermetall- und Erdalkalimetallionen für die verschiedensten technischen Einsatzgebiete bereitzustellen, die neben guten komplexbildenden Eigenschaften ökologisch unbedenklich, d.h. biologisch gut abbaubar sind.

Demgemäß wurden die eingangs definierten 2-Methyl- und 2-Hydroxymethyl-serin-N,N-diessigsäure und ihre Derivate I gefunden.

Die Verbindungen I können als freie Carbonsäuren (X = H) oder in einer partiellen oder vollständigen Salzform auftreten. Im letzteren Fall steht X dann für Alkalimetallionen wie Lithium oder insbesondere Natrium oder Kalium oder für das Ammoniumion, das partiell oder vollständig durch $C_1$-$C_4$-Alkylgruppen oder $C_1$-$C_4$-Hydroxyalkylgruppen substituiert sein kann. Insbesondere sind hier Triaminsalze mit tertiärem Stickstoffatom zu nennen, denen Aminbasen wie Trimethylamin, Triethylamin, Tri-n-propylamin, Triisopropylamin, Tri-n-butylamin oder Triisobutylamin sowie Trialkanolamine, beispielsweise Triethanolamin oder Triisopropanolamin, zugrundeliegen.

Die Verbindungen I werden zweckmäßigerweise nach einer der vier nachfolgend beschriebenen Ausführungsformen (a) bis (d) hergestellt.

Bei Ausführungsform (a) werden Amine der allgemeinen Formel II

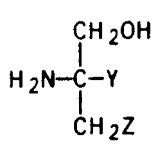

II

in der Y die Gruppe COOR, $CONH_2$ oder CN bedeutet, wobei R für Wasserstoff, ein Alkalimetall wie Natrium oder Kalium, Ammonium oder einen $C_1$-$C_4$-Alkylrest, beispielsweise Methyl, Ethyl, n-Propyl, iso-Propyl, n-Butyl oder tert.-Butyl, steht, mit Formaldehyd und Cyanwasserstoff oder Aklalimetallcyaniden, z.B. Natriumcyanid oder Kaliumcyanid, zu 2-Methyl- bzw. 2-Hydroxymethyl-serin-N,N-diacetonitril oder seinen Derivaten der allgemeinen Formel V umgesetzt:

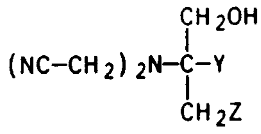

V

Die bevorzugte Ausgangsverbindung II ist 2-Methyl- bzw. 2-Hydroxymethyl-serin in Form seines racemischen Gemisches, vorteilhaft kann auch das Natrium-, Kalium- oder Ammoniumsalz hiervon eingesetzt werden.

Die Umsetzung erfolgt in an sich bekannter Weise nach Art der Strecker' schen Synthese, wie sie beispielsweise in Houben-Weyl: Methoden der organischen Chemie, Bd. 11/2, S. 408-412, 1958, Thieme-Verlag Stuttgart, für Diamine, Carbonylverbindungen und wäßrige Cyanwasserstoff-Lösungen beschrieben ist.

Die Umsetzung wird bevorzugt in Wasser, aber auch in einem organischen Lösungsmittel oder in Mischungen hieraus durchgeführt. Als organische Lösungsmittel kommen vorzugsweise solche zum Einsatz, die mit Wasser teilweise oder vollständig mischbar sind, z.B. Methanol, Ethanol, n-Propanol, iso-Propanol, tert.-Butanol, Dioxan oder Tetrahydrofuran.

Man setzt zweckmäßigerweise pro Mol II 2 bis 2,6 mol Formaldehyd, vorzugsweise in Form seiner wäßrigen ca. 30 gew.-%igen Lösung, und 2 bis 2,3 mol Cyanwasserstoff oder Alkalimetallcyanid ein. Die Umsetzung wird normalerweise im Falle von wasserfreiem Cyanwasserstoff bei Temperaturen von 0 bis 45 °C, insbesondere 15 bis 25 °C, und im Falle von Alkalimetallcyaniden bei 40 bis 100 °C, insbesondere 70 bis 100 °C, vorgenommen. Für die Umsetzung mit wasserfreiem Cyanwasserstoff kommt in der Regel ein pH-Bereich von 0 bis 11, insbesondere von 3 bis 9, in Betracht.

An die Umsetzung von II zum Zwischenprodukt V schließt sich zweckmäßigerweise eine Hydrolyse noch vorhandener Carbonsäureamid- und Nitrilgruppen zu Carboxylgruppen an, welche in an sich bekannter Weise in wäßrigem Reaktionsmedium in Gegenwart von Basen wie Natron- oder Kalilauge oder von Säuren wie Schwefel- oder Salzsäure bei Temperaturen von 20 bis 110 °C, insbesondere 40 bis 100 °C, durchgeführt wird.

Entsprechend den Reaktionsbedingungen erhält man die Verbindungen I als freie Carbonsäuren oder beispielsweise als Alkalimetallsalze. Aus den freien Säuren können durch Neutralisation mit den entsprechenden Basen, beispielsweise Aminbasen, die gewünschten Salze I ohne Schwierigkeit hergestellt werden.

Die Verbindungen I lassen sich aus ihren Lösungen problemlos in reiner Form isolieren. Hierfür bieten sich insbesondere Sprüh- oder Gefriertrocknung, Kristallisation und Fällung an. Es kann vorteilhaft sein, die bei der Herstellung angefallene Lösung direkt einer technischen Verwendung zuzuführen.

Bei Ausführungsform (b) werden Amin II in an sich bekannter Weise mit einer Monohalogenessigsäure oder ihren $C_1$-$C_4$-Alkylestern oder einem Monohalogenacetonitril zu V oder den hierzu analogen Verbindungen umgesetzt. Als Monohalogenessigsäure und Derivate hiervon kommen in Betracht: Chloressigsäure, Chloressigsäuremethylester, Chloressigsäureethylester, Chloressigsäurepropylester,Chloressigsäurebutylester, Chloracetonitril sowie die analogen Bromverbindungen.

Man arbeitet wie bei Ausführungsform (a) bevorzugt in Wasser, aber auch in einem organischen Lösungsmittel oder in Mischungen hieraus. Es werden zweckmäßigerweise pro Mol II 2 bis 2,6 mol der Monohalogenessigsäure oder eines ihrer Derivate bei Temperaturen von 0 bis 100 °C, insbesondere 40 bis 80 °C, und einem pH-Wert von 7 bis 14, insbesondere 7, 5 bis 12, mit dem Amin II zur Reaktion gebracht. Die Umsetzung wird in der Regel in natron- oder kalialkalischer wäßriger Lösung oder in Gegenwart eines Säurefängers, beispielsweise eines tertiären Amins wie Trimethylamin, Triethylamin, Tri-n-propylamin, Triisopropylamin, Tri-n-butylamin, Triisobutylamin oder 1,4-Diazabicyclo[2.2.2]octan, vorgenommen.

Die sich anschließende Hydrolyse noch vorhandener Carbonsäureamid-, Carbonsäureester- und Nitrilgruppen sowie die weitere Verarbeitung der so erhaltenen Verbindungen I erfolgt zweckmäßigerweise wie bei Ausführungsform (a).

Bei Ausführungsform (c) wird Hydroxyaceton oder 1,3-Dihydroxyaceton mit Iminoverbindungen der allgemeinen Formel III

$(Y\text{-}CH_2)_2NH$ III

und Cyanwasserstoff oder Alkalimetallcyaniden in an sich bekannter Weise zu den Serinnitril-Derivaten der allgemeinen Formel Va umgesetzt:

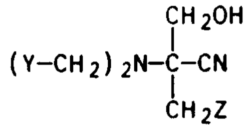

Va

Als Iminoverbindungen III können beispielsweise Iminodiessigsäure, ihre Mono- oder Dinatrium-, -kalium- oder -ammoniumsalze, Iminodiacetamid, Iminodiacetonitril, Iminodiessigsäuremono- oder -dimethylester und -mono- oder -diethylester eingesetzt werden.

Die Umsetzung wird wie bei Ausführungsform (a) bevorzugt in Wasser, aber auch in einem organischen Lösungsmittel oder in Mischungen hieraus durchgeführt. Man setzt das Hydroxy- bzw. Dihydroxyaceton, die Iminoverbindung III und Cyanwasserstoff bzw. Cyanid zweckmäßigerweise in äquimolarem oder annähernd äquimolarem Verhältnis ein. Man arbeitet normalerweise bei Temperaturen von 10 bis 100 °C, insbesondere 10 bis 60 °C, und einem pH-Wert von 0 bis 13, insbesondere von 0,5 bis 9.

In einer bevorzugten Ausführungsform von (c) wird das Hydroxy- bzw. Dihydroxyaceton zuerst mit der Iminoverbindung III und danach mit Cyanwasserstoff bzw. Cyanid zur Reaktion gebracht. Die Reihenfolge der Schritte kann aber auch vertauscht werden.

Die sich anschließende Hydrolyse noch vorhandener Carbonsäureamid-, Carbonsäureester- und Nitrilgruppen im Zwischenprodukt Va sowie die weitere Verarbeitung der so erhaltenen Verbindungen I erfolgt zweckmäßigerweise wie bei Ausführungsform (a).

Für den Fall, daß Z eine Hydroxylgruppe bezeichnet, kann auch gemäß Ausführungsform (d) Nitrilotriacetonitril mit Formaldehyd in an sich bekannter Weise umgesetzt werden.

Die Umsetzung wird bevorzugt in einem niederen Alkohol wie Methanol, Ethanol, Isopropanol und n-Butanol oder in Wasser durchgeführt, daneben können aber auch Ether wie Tetrahydrofuran und Dioxan oder Mischungen aller genannter Lösungsmittel eingesetzt werden. Man setzt pro Mol Nitrilotriacetonitril in der Regel 2 bis 5 mol, vorzugsweise 2 bis 3 mol Formaldehyd, vorzugsweise in Form seiner wäßrigen ca. 30 gew.-%igen Lösung, ein. Man arbeitet normalerweise in Gegenwart von Basen bei einem pH-Wert von 7,5 bis 12, insbesondere 8,5 bis 11, und bei Temperaturen von 0 bis 100 °C, insbesondere 25 bis 80 °C.

Geeignete Basen sind z.B. tertiäre aliphatische Amine, insbesondere Trialkylamine wie Triethylamin und Trialkanolamin wie Triethanolamin, Erdalkalimetallhydroxide wie Calcium- und Magnesiumhydroxid, Alkalimetallhydroxide wie Natrium- und Kaliumhydroxid, Alkalimetallcarbonate wie Natrium- und Kaliumcarbonat sowie stark basische Kunstharz-Anionenaustauscher in der OH-Form.

Die sich anschließende Hydrolyse der Nitrilgruppen im so erhaltenen Zwischenprodukt V (Y = CN, Z = OH) sowie die weitere Verarbeitung des resultierenden Produktes I erfolgt zweckmäßigerweise wie bei Ausführungsform (a).

Die erfindungsgemäßen Verbindungen I sind in hervorragender Weise dazu geeignet, Schwermetall- oder Erdalkalimetallionen wie beispielsweise Eisen, Kupfer, Mangan, Zink, Calcium oder Magnesium oder Gemische solcher Ionen zu komplexieren. Aufgrund dieser Fähigkeit weisen sie eine Vielzahl von technischen Anwendungsmöglichkeiten auf. Da es sich bei den erfindungsgemäßen Verbindungen I um biologisch abbaubare Substanzen handelt, können sie überall dort vorteilhaft eingesetzt werden, wo bei der Anwendung Abwasser in größeren Mengen anfällt und erst geklärt werden muß, bevor es in die Oberflächengewässer gelangt.

Als Einsatzgebiete und Verwendungszwecke kommen beispielsweise Wasch- und Reinigungsmittel, technische Anwendungen als Industriereiniger, in der Galvanotechnik, in der Wasserbehandlung und bei Polymerisationen, die photographische Industrie, die Textilindustrie und die Papierindustrie sowie verschiedene Anwendungen in Pharmazeutika, in der Kosmetik, bei Nahrungsmitteln und bei der Pflanzenernährung in Betracht.

Ihre vorteilhafte Wirkung liegt auch in einer Bleichmittelstabilisierung, beispielsweise für Natriumperborate wie $NaBO_2 \cdot H_2O_2 \cdot 3\ H_2O$, Peroxycarbonate, Peroxyphosphonate, Citratperhydrate, Harnstoff- und Melamin-$H_2O_2$-Addukte, Caroate, Perbenzoate, Peroxyphthalate und Alkalimetallhypochlorite in Wasch- und Reinigungsmitteln und bei der Wasserstoffperoxidbleiche von Textilien, Zellstoff oder Papierrohstoff. Spuren von Schwermetallen wie Eisen, Kupfer und Mangan, kommen im Waschpulver selbst, im Wasser und im Textilgut vor und katalysieren die Zersetzung der Perverbindungen oder des daraus entstandenen Wasserstoffperoxids. Die erfindungsgemäßen Komplexbildner I binden diese Metallionen und verhindern die unerwünschte Zersetzung des Bleichsystems während der Lagerung und in der Waschflotte. Dadurch erhöht sich die Effizienz des Bleichsystems und Faserschädigungen werden zurückgedrängt.

In flüssigen Waschmittelformulierungen können die neuen Komplexbildner I als sog. Konservierungsmittel, zweckmäßigerweise in einer Menge von 0,05 bis 1 Gew.-%, bezogen auf das Gesamtgewicht der Waschmittelformulierung, eingesetzt werden.

In Seifen verhindern die neuen Komplexbildner I beispielsweise metallkatalysierte oxidative Zersetzungen.

Weiterhin dienen sie in hervorragender Weise in Wasch- und Reinigungsmitteln als Gerüststoff (Builder), um Ausfällungen und Inkrustationen auf dem Gewebe zu verhindern.

Sie können in vorteilhafter Weise überall dort eingesetzt werden, wo bei technischen Verfahren Ausfällungen von Calcium-, Magnesium- und Schwermetallsalzen stören und verhindert werden sollen, beispielsweise zur Verhinderung von Ablagerungen und Verkrustungen in Kesseln, Rohrleitungen, an Sprühdüsen oder allgemein an glatten Oberflächen.

Sie können zur Stabilisierung von Phosphaten in alkalischen Entfettungsbädern und Verhinderung der Ausfällung von Kalkseifen dienen und verhindern dadurch das "Anlaufen" von Nichteisenoberflächen und verlängern die Standzeiten von alkalischen Reinigerbädern.

Sie können als Komplexbildner in alkalischen Entrostungs- und Entzunderungsbädern verwendet werden sowie in galvanischen Bädern anstelle von Cyaniden.

Die Kühlwasserbehandlung mit den neuen Komplexbildnern I verhindert Ablagerungen bzw. löst bereits vorhandene wieder auf. Ein besonderer Vorteil ist die Anwendungsmöglichkeit in alkalischem Medium und damit die Beseitigung von Korosionsproblemen.

Bei der Polymerisation von Kautschuk können sie zur Herstellung der dabei verwendeten Redoxkatalysatoren verwendet werden. Sie verhindern zusätzlich das Ausfallen von Eisenhydroxid im alkalischen Polymerisationsmilieu.

In der photographischen Industrie können die neuen Komplexbildner in Entwickler- oder Fixierbädern, die mit hartem Wasser angesetzt werden, verwendet werden, um die Ausfällung schwerlöslicher Calcium- und Magnesium-Salze zu verhindern. Die Ausfällungen führen zur Grauschleiern auf Filmen und Bildern sowie Ablagerungen in den Tanks, die somit vorteilhaft vermieden werden können. Sie können als Eisen-III-Komlexbildnerlösungen vorteilhaft in Bleich- und Bleichfixierbädern eingesetzt werden und so die aus ökologischen Gründen bedenklichen Hexacyanoferratlösungen ersetzen.

In der Textilindustrie können sie zur Entfernung von Schwermetallspuren während des Herstellungs- bzw. Färbeprozesses von natürlichen und synthetischen Fasern dienen. Dadurch werden viele Störungen verhindert, beispielsweise Schmutzflecken und Streifen auf dem Textilgut, Verlust des Glanzes, schlechte Benetzbarkeit, unegale Färbungen und Farbfehler.

In der Papierindustrie können sie zur Eliminierung von Schwermetallionen, insbesondere Eisenionen, verwendet werden. Die Ablagerung von Eisen auf Papier führt zu "heißen Flecken", an denen die oxidative katalytische Zerstörung der Zellulose beginnt. Ferner katalysieren Schwermetallionen die Zersetzung von $H_2O_2$, welches für die Papierbleichung eingesetzt wird.

Weiterhin kommen beispielsweise Anwendungen in Pharmazeutika, Kosmetika und Nahrungsmitteln in Betracht, um die metallkatalysierte Oxidation von olefinischen Doppelbindungen und damit das Ranzigwerden der Erzeugnisse zu verhindern.

In der Pflanzenernährung werden zur Behebung von Schwermetalldefiziten Kupfer-, Eisen-, Mangan- und Zink-Komplexe mit I verwendet. Diese Schwermetalle werden als Chelate zugegeben, um die Ausfällung als biologisch inaktive unlösliche Salze zu verhindern.

Weitere Anwendungsgebiete für die neuen Komplexbildner I sind die Rauchgaswäsche, und zwar die gleichzeitige Entfernung von $NO_x$ und $SO_2$ aus Rauchgasen, die Entschwefelung nach Wellman-Lord, die $H_2S$-Oxidation, die Metallextraktion sowie Anwendungen als Katalysatoren für organische Synthesen, z.B. Luftoxidation von Paraffinen und Hydroformylierung von Olefinen zu Alkoholen.

Die erfindungsgemäßen Verbindungen I mit ihren hervorragenden komplexbildenden Eigenschaften können unter den genannten Anwendungsgebieten in ganz besonderem Maße als Bleichmittelstabilisatoren und als Gerüststoffe in Wasch- und Reinigungsmitteln empfohlen werden.

Gegenstand der vorliegenden Erfindung sind auch Mittel zur Komplexierung von Schwermetall- oder Erdalkalimetallionen oder Gemischen hiervon, die je nach Anwendungszweck die erfindungsgemäßen Verbindungen I in einer Menge von 0,01 bis 99 Gew.-%, bezogen auf die Gesamtmenge der Zubereitungen, enthalten.

Weiterhin sind Gegenstand der vorliegenden Erfindung Wasch- und Reinigungsmittel, die 0,01 bis 20 Gew.-%, vorzugsweise 0,05 bis 10 Gew.-%, bezogen auf die Gesamtmenge der Zubereitung, einer oder mehrerer erfindungsgemäßer Verbindungen I enthalten. Bei einer Verwendung bevorzugt als Gerüststoff sind Mengen von 1 bis 10 Gew.-%, bei einer Verwendung bevorzugt als Bleichmittelstabilisator beispielsweise für Perborate sind Mengen von 0,05 bis 1 Gew.-% besonders bevorzugt. Bei einer Verwendung insbesondere als Komplexbildner in Waschmitteln sind Mengen von 0,1 bis 2 Gew.-% bevorzugt.

Die erfindungsgemäßen Verbindungen I können in ihrer Eigenschaft als Komplexbildner, Gerüststoff und Bleichmittelstabilisator in Wasch- und Reinigungsmittelformulierungen auch zusammen mit anderen Mitteln des Standes der Technik verwendet werden, wobei die allgemeinen Eigenschaften im Hinblick auf Sequestrierung, Inkrustationsinhibierung, Vergrauungsinhibierung, Primärwaschwirkung und Bleichwirkung unter Umständen deutlich verbessert werden können.

Wasch- und Reinigungsmittelformulierungen mit den erfindungsgemäßen Verbindungen I enthalten in der Regel als zusätzliche Bestandteile, bezogen auf das Gesamtgewicht, 6 bis 25 Gew.-% Tenside, 15 bis 50 Gew.-% Builder und gegebenenfalls Co-Builder und 5 bis 30 Gew.-% Hilfsstoffe wie Enzyme, Schaumregulatoren, Korrosionsinhibitoren, optische Aufheller, Duftstoffe, Farbstoffe oder Formulierhilfsmittel wie z.B. Natriumsulfat. Die genaue

Spezifikation dieser zusätzlichen Bestandteile ist dem Fachmann bekannt und braucht deshalb hier nicht näher ausgeführt werden.

Die erfindungsgemäße 2-Methyl- und 2-Hydroxymethyl-serin-N,N-diessigsäure und ihre Derivate I haben hervorragende komplexbildende Eigenschaften und sind hierin handelsüblichen Komplexbildnern wie Nitrilotriessigsäure und Ethylendiamintetraessigsäure mindestens ebenbürtig. Auch in ihren Eigenschaften als Gerüststoff in Wasch- und Reinigungsmitteln zur Verbesserung der Weißwaschwirkung und zur Verhinderung von Ablagerungen auf dem Gewebe sind die Verbindungen I mit beispielsweise Nitrilotriessigsäure oder Ethylendiamintetraessigsäure vergleichbar. Eine deutlich überlegene Wirkung zeigt sich jedoch bei der Bleichmittelstabilisierung. Ein weiterer Vorzug ist die biologische Abbaubarkeit der Verbindungen I.

## Beispiele

Die Prozentangaben beziehen sich, wenn nicht anders angegeben, auf das Gewicht.

### Beispiel 1

Herstellung von 2-Hydroxymethyl-serin-N,N-diessigsäure gemäß Ausführungsform (a)

Zu 100 g einer 30 %igen wäßrigen Formaldehydlösung (entsprechend 1,0 mol $CH_2O$) wurde innerhalb von 75 Minuten bei 20 bis 25 °C eine Lösung von 67 g 2-Hydroxymethyl-serin (entsprechend 0,5 mol) in 250 g Wasser, die zuvor durch Zugabe von 37 g 40 %iger wäßriger Natronlauge auf einen pH-Wert von 8,5 eingestellt worden war, getropft. Nach 30minütigem Nachrühren bei der angegebenen Temperatur wurde die Lösung auf 15 bis 20 °C abgekühlt und innerhalb von 90 Minuten mit 27 g wasserfreiem Cyanwasserstoff (entsprechend 1,0 mol) tropfenweise versetzt. Anschließend wurde 30 Minuten bei 30 °C nachgerührt.

Die so hergestellte Lösung von 2-Hydroxymethyl-serin-N,N-diacetonitril wurde innerhalb von 1 Stunde bei 80 bis 100 °C zu 101 g 40 %iger wäßriger Natronlauge getropft. Nach 3stündigem Nachrühren bei 100 °C wurde keine Ammoniakentwicklung mehr festgestellt. Es resultierten 487 g einer klaren, gelblichen, 30 %igen wäßrigen Lösung des Trinatriumsalzes von 2-Hydroxymethyl-serin-N,N-diessigsäure.

Aus der erhaltenen Trinatriumsalz-Lösung wurde im Wasserstrahlvakuum solange Wasser abdestilliert, bis die Lösung einen Feststoffgehalt von ca. 50 % aufwies. Danach wurde mit Bromwasserstoffsäure ein pH-Wert von 2 eingestellt. Diese Lösung wurde in die 4fache Volumenmenge Methanol eingegossen, der entstandene Niederschlag abfiltriert und nochmals mit Methanol gewaschen. Nach Trocknung erhielt man 98 g 2-Hydroxymethyl-serin-N,N-diessigsäure (entsprechend 78 % der Theorie, bezogen auf 2-Hydroxymethyl-serin).

### Beispiel 2

Herstellung von 2-Hydroxymethyl-serin-N,N-diessigsäure gemäß Ausführungsform (c)

Zu einer Lösung von 45 g 1,3-Dihydroxyaceton (entsprechend 0,5 mol) in 100 g Wasser wurde bei 25 °C innerhalb von 30 Minuten eine Lösung von 66,6 g Iminodiessigsäure (entsprechend 0,5 mol) in 120 g Wasser, die zuvor durch Zugabe von 50 g 40 %iger wäßriger Natronlauge auf einen pH-Wert von 7 eingestellt worden war, getropft. Anschließend wurde die Lösung auf 15 bis 20 °C abgekühlt und innerhalb von 45 Minuten mit 13,6 g wasserfreiem Cyanwasserstoff (entsprechend 0,5 mol) tropfenweise versetzt. Danach wurde 5 Stunden bei 30 °C nachgerührt.

Die so hergestellte Lösung von 2-Hydroxymethyl-serinnitril-N,N-diessigsäurewurde mit 90 g 40 %iger wäßriger Natronlauge versetzt und 4 Stunden auf 90 °C erhitzt. Danach wurde kein Ammoniakgeruch mehr festgestellt.

Aus der resultierenden orangefarbenen Lösung des Trinatriumsalzes der 2-Hydroxymethyl-serin-N,N-diessigsäure wurde die freie Säure analog Beispiel 1 in einer Ausbeute von 60 %, der Theorie, bezogen auf 1,3-Dihydroxyaceton, hergestellt.

Beispiel 3

Herstellung von 2-Hydroxymethyl-serin-N,N-diessigsäure gemäß Ausführungsform (d)

Zu einer Lösung von 134 g Nitrilotriacetonitril (entsprechend 1,0 mol) in 450 g Ethanol, die durch Zugabe von Triethylamin auf einen pH-Wert von 9,5 (gemessen an einer Probe in 10 %iger wäßriger Lösung) eingestellt worden war, wurden bei 75 °C innerhalb von 4 Stunden 250 g einer 30 %igen wäßrigen Formaldehydlösung (entsprechend 2,5 mol $CH_2O$) unter Konstanthalten des pH-Wertes getropft. Nach 4stündigem Nachrühren bei der angegebenen Temperatur wurde die erhaltene Lösung von 2, 2-Dihydroxymethyl-nitrilotriacetonitril zu 300 g einer auf 100 °C erhitzten 40 %igen wäßrigen Natronlauge innerhalb von 30 Minuten getropft. Nach weiteren 4 Stunden bei 100 °C entwich kein Ammoniak mehr.

Aus der resultierenden Lösung des Trinatriumsalzes der 2-Hydroxymethyl-serin-N,N-diessigsäure wurde die freie Säure analog Beispiel 1 in einer Ausbeute von 51 % der Theorie, bezogen auf Nitrilotriacetonitril, hergestellt.

Beispiel 4

Herstellung von 2-Methylserin-N,N-diessigsäure gemäß Ausführungsform (a)

Zu 100 g einer 30 %igen wäßrigen Formaldehydlösung (entsprechend 1,0 mol $CH_2O$) wurde innerhalb von 75 Minuten bei 20 bis 25 °C eine Lösung von 59 g 2-Methylserin (entsprechend 0,5 mol) in 250 ml Wasser, die zuvor durch Zugabe von 37 g 40 %iger wäßriger Natronlauge auf einen pH-Wert von 8,5 eingestellt worden war, getropft. Nach 30minütigem Nachrühren bei der angegebenen Temperatur wurde die Lösung auf 15 bis 20 °C abgekühlt und innerhalb von 90 Minuten mit 27 g wasserfreiem Cyanwasserstoff (entsprechend 1,0 mol) tropfenweise versetzt. Anschließend wurde 30 Minuten bei 30 °C nachgerührt.

Die so erhaltene Lösung von 2-Methylserin-N,N-diacetonitril wurde innerhalb von 1 Stunde bei 80 bis 100 °C zu 102 g 40 %iger wäßriger Natronlauge getropft. Nach 3stündigem Nachrühren bei 100 °C wurde keine Ammoniakentwicklung mehr festgestelt. Es resultierten 472 g einer klaren, gelblichen, 30 %igen wäßrigen Lösung des Trinatriumsalzes von 2-Methylserin-N,N-diessigsäure.

Aus der so hergestellten Trinatriumsalz-Lösung wurde im Wasserstrahlvakuum solange Wasser abdestilliert, bis die Lösung einen Feststoffgehalt von ca. 50 % aufwies. Danach wurde mit konzentrierter Salzsäure ein pH-Wert von 2 eingestellt. Die Lösung wurde in die 4fache Volumenmenge Methanol eingegossen, der entstandene Niederschlag abfiltriert und nochmals mit Methanol gewaschen. Nach Trocknung erhielt man 96 g 2-Methylserin-N,N-diessigsäure (entsprechend 82 % der Theorie, bezogen auf 2-Methylserin).

Beispiel 5

Herstellung von 2-Methylserin-N,N-diessigsäure gemäß Ausführungsform (c)

Zu einer Lösung von 37 g Hydroxyaceton (entsprechend 0,5 mol) in 100 g Wasser wurde bei 25 °C innerhalb von 30 Minuten eine Lösung von 66,6 g Iminodiessigsäure (entsprechend 0,5 mol) in 120 g Wasser, die zuvor durch Zugabe von 50 g 40 %iger wäßriger Natronlauge auf einen pH-Wert von 7 eingestellt worden war, getropft. Anschließend wurde die Lösung auf 15 bis 20 °C abgekühlt und innerhalb von 45 Minuten mit 13,6 g wasserfreiem Cyanwasserstoff (entsprechend 0,5 mol) tropfenweise versetzt. Danach wurde 5 Stunden bei 30 °C nachgerührt.

Die so hergestellte Lösung von 2-Methylserinnitril-N,N-diessigsäure wurde mit 90 g 40 %iger wäßriger Natronlauge versetzt und 4 Stunden auf 90 °C erhitzt. Danach wurde kein Ammoniakgeruch mehr festgestellt.

Aus der resultierenden orangefarbenen Lösung des Trinatriumsalzes der 2-Methylserin-N,N-diessigsäure wurde die freie Säure analog Beispiel 1 in einer Ausbeute von 61 % der Theorie, bezogen auf Hydroxyaceton, hergestellt.

Anwendungstechnische Eigenschaften

Als anwendungstechnische Eigenschaften der erfindungsgemäßen 2-Methyl- und 2-Hydroxymethylserin-N,N-diessigsäure und ihrer Derivate I wurde das Calcium-Bindevermögen sowie die Natriumperboratstabilisierung und die Inkrustationsinhibierung in Waschmittelformulierungen ermittelt.

A) Bestimmung des Calcium-Bindevermögens

Die inhibierende Wirkung von Komplexbildnern oder Dispergiermitteln auf die Ausfällung von Calciumcarbonat wird durch Trübungstitration bestimmt. Die zu untersuchende Substanz wird in Gegenwart von Natriumcarbonat mit Calciumacetatlösung titriert. Der Endpunkt wird durch Bildung des Calciumcarbonat-Niederschlages angezeigt. Durch Verwendung einer ausreichenden Menge an Natriumcarbonat wird sichergestellt, daß die Messung auch dann ein korrektes Ergebnis liefert, wenn die Wirkung nicht nur auf einer Komplexierung der Calciumionen beruht, sondern auf der Dispergierung von Calciumcarbonat.

Während der Titration wird die Änderung der Lichtdurchlässigkeit mit Hilfe eines Lichtleiterphotometers verfolgt. Bei letzterem wird ein über Glasfaser in die Lösung geleiteter Lichtstrahl an einem Spiegel reflektiert und die Intensität des reflektierten Lichts gemessen.

Zur Durchführung der Bestimmung wurde 1,0 g 2-Hydroxymethyl-serin-N,N-diessigsäure bzw. 1,0 g 2-Methylserin-N,N-diessigsäure in 100 ml destilliertem Wasser gelöst und mit 1 normaler wäßriger Natronlauge ein pH-Wert von 10 eingestellt. Anschließend wurden 10 ml 10 %ige Natriumcarbonat-Lösung zugegeben. Unter Konstanthalten eines pH-Wertes von 11 wurde die Probelösung bei 20 °C mit 0,25 molarer wäßriger Calciumacetat-Lösung automatisch titriert. Die Titration wurde bei einem pH-Wert von 10 und einer Temperatur von 80 °C wiederholt. Als Ergebnis wurde in der unten angeführten Tabelle jeweils die komplexierte Menge an Calciumcarbonat in mg pro g des Komplexbildners angegeben, wobei sich diese Menge nach der Gleichung

mg $CaCO_3$/g Komplexbildner = Verbrauch an $Ca(OAc)_2$-Lösung in ml x 25

errechnet.

B) Bestimmung der Natriumperboratstabilisierung in Waschflotten

Das für die Bleichwirkung in natriumperborathaltigen Waschmittelformulierungen verantwortliche Wasserstoffperoxid wird durch Schwermetallionen (Fe, Cu, Mn) katalytisch zersetzt. Durch Komplexierung der Schwermetallionen läßt sich dieses verhindern. Die peroxidstabilisierende Wirkung der Komplexbildner wird über den Restperoxidgehalt nach Warmlagerung einer schwermetallhaltigen Waschflotte geprüft.

Der Gehalt an Wasserstoffperoxid wurde vor und nach der Lagerung der Waschflotte bei 60 °C oder 80 °C durch Titration mit Kaliumpermanganat in saurer Lösung bestimmt. Als Ergebnis wurde in der unten angeführten Tabelle jeweils der Prozentsatz an nach der Warmlagerung noch vorhandenem $H_2O_2$ angegeben.

Zur Prüfung auf Perboratstabilisierung wurden zwei Waschmittelformulierungen (1) und (2) benutzt, wobei die Zersetzung bei der Warmlagerung durch Zugabe von Schwermetallkatalysatoren (2,5 ppm Mischung aus 2 ppm $Fe^{3+}$, 0,25 ppm $Cu^{2+}$ und 0,25 ppm $Mn^{2+}$) erfolgte:

(1) Zusammensetzung der phosphatreichen Formulierung:
19,3 % Natrium-$C_{12}$-Alkylbenzolsulfonat (50 %ige wäßrige Lösung)
15,4 % Natriumperborat-Tetrahydrat
30,8 % Natriumtriphosphat
2,6 % Copolymer aus Maleinsäure und Acrylsäure (Gewichtsverhältnis 50:50, mittlere Molmasse 50.000)
31,0 % wasserfreies Natriumsulfat
0,9 % erfindungsgemäßer Komplexbildner oder Vergleichssubstanz
Die Waschmittelkonzentration betrug 6,5 g/l unter Verwendung von Wasser mit 25 °dH. Die Lagerung erfolgte 2 Stunden bei 80 °C.

(2) Zusammensetzung der phosphatarmen Formulierung:
15 % Natrium-$C_{12}$-Alkylbenzolsulfonat (50 %ige wäßrige Lösung)
5 % Anlagerungsprodukt von 11 Mol Ethylenoxid an 1 Mol Talgfettalkohol
20 % Natriumperborat-Tetrahydrat
6 % Natriummetasilicat • 5 $H_2O$
1,25 % Magnesiumsilicat
20 % Natriumtriphosphat
31,75 % wasserfreies Natriumsulfat
1 % erfindungsgemäßer Komplexbildner oder Vergleichssubstanz
Die Waschmittelkonzentration betrug 8 g/l unter Verwendung von Wasser mit 25 °dH. Die Lagerung erfolgte 1 Stunde bei 60 °C.

C) Bestimmung der Inkrustationsinhibierung

Ein Maß für die beim Waschprozeß auf dem Textilgewebe entstandenen Ablagerungen der Härtebildner ist der Gewichtsanteil an Asche, der beim Veraschen des gewaschenen Testgewebes zurückbleibt. Die den Waschmittelformulierungen zugesetzten Gerüststoffe (Builder) haben die Aufgabe, den Anteil dieser Inkrustation gering zu halten.

Die in der unten aufgeführten Tabelle genannten Prozentgehalte an Asche, bezogen auf das trockene Testgewebe vor dem Waschen, wurden nach den üblichen Veraschungs-Verfahren im Muffelofen bei 700 °C ermittelt.

Zur Untersuchung der Inkrustierung wurden folgende Versuchsbedingungen gewählt:

| | |
|---|---|
| Gerät: | Launder-O-meter der Firma Atlas, Chicago, US |
| Zahl der Waschzyklen: | 15 |
| Waschflotte: | 250 ml, wobei das verwendete Wasser 4 mMol Härte pro Liter aufwies (Ca/Mg 4:1) |
| Waschdauer: | 30 Minuten auf 60 °C (einschließlich Aufheizzeit) |
| Waschmitteldosierung: | 8 g/l |
| Prüfgewebe | 10 g Baumwolle und<br>10 g Baumwollfrottee |

Es wurde eine Waschmittelformulierung der folgenden Zusammensetzung verwendet:

12,5 % Natrium-$C_{12}$-Alkylbenzolsulfonat (50 %ige wäßrige Lösung)
4,7 % Anlagerungsprodukt von 7 Mol Ethylenoxid an 1 Mol $C_{13}$/$C_{15}$-Oxoalkohol
2,8 % einer handelsüblichen Seife
25 % eines handelsüblichen Zeoliths A
5 % Natriumsalz eines Copolymeren aus Acrylsäure und Maleinsäure (Gewichtsverhältnis 70:30, mittlere Molmasse 70.000)
4 % Natriumdisilicat
1 % Magnesiumsilicat
20 % Natriumperborat-Tetrahydrat
16 % wasserfreis Natriumsulfat
1 % Tylose
8 % erfindungsgemäßer Komplexbildner oder Vergleichssubstanz in Form des Natriumsalzes

Die nachfolgende Tabelle zeigt die Ergebnisse der Prüfungen A bis C, wobei die erfindungsgemäßen Komplexbildner 2-Hydroxymethyl-serin-N,N-diessigsäure (Ia) und 2-Methylserin-N,N-diessigsäure (Ib), gegebenenfalls in ihrer Trinatriumsalzform, in ihren Eigenschaften mit den handelsüblichen Komplexbildnern Nitrilotriessigsäure (NTA) und Ethylendiamintetraessigsäure (EDTA) vergleichen wurden.

Dabei erreichen die erfindungsgemäßen Verbindungen Ia und Ib bezüglich Calcium-Bindevermögen und Inkrustationsinhibierung ähnliche Werte wie NTA und EDTA. Bei der Natriumperboratstabilisierung sind sie jedoch den handelsüblichen Komplexbildnern deutlich überlegen.

Tabelle

| Komplex-bildner | Prüfung A Calcium-Bindevermögen [mg $CaCO_3$/g Komplexbildner] | | Prüfung B Natriumperboratstabilisierung [Rest-% $H_2O_2$] Formulierungen | | Prüfung C Inkrustationsinhibierung [% Asche] | |
|---|---|---|---|---|---|---|
| | bei 20°C/pH 11 | bei 80°C/pH 10 | (1) | (2) | Baumwolle | Baumwollfrottee |
| Ia | 265 | 250 | 76 | 83 | 0,47 | 0,69 |
| Ib | 320 | 270 | 81 | 83 | 0,54 | 0,54 |
| NTA* | 405 | 435 | 24,5 | 32,5 | 0,49 | 0,54 |
| EDTA* | 315 | 320 | 20 | 34 | (nicht gemessen) | |
| ohne | - | - | - | - | 0,74 | 2,33 |

* zum Vergleich

## Patentansprüche

1. 2-Methyl- und 2-Hydroxymethyl-serin-N,N-diessigsäure und ihre Derivate der allgemeinen Formel I

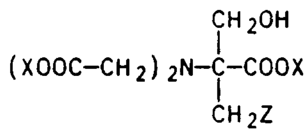

I

in der X für Wasserstoff, Alkalimetalle oder Ammonium, das durch $C_1$-$C_4$-Alkylgruppen oder $C_1$-$C_4$-Hydroxyalkylgruppen substituiert sein kann, steht und Z Wasserstoff oder eine Hydroxylgruppe bezeichnet.

2. Verfahren zur Herstellung von 2-Methyl- und 2-Hydroxymethyl-serin-N,N-diessigsäure und ihren Derivaten I gemäß Anspruch 1, dadurch gekennzeichnet, daß man
(a) Amine der allgemeinen Formel II

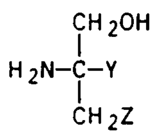

II

in der Y die Gruppe COOR, $CONH_2$ oder CN bedeutet, wobei R für Wasserstoff, ein Alkalimetall, Ammonium oder einen $C_1$-$C_4$-Alkylrest steht, in an sich bekannter Weise mit Formaldehyd und Cyanwasserstoff oder Alkalimetallcyaniden umsetzt und anschließend noch vorhandene Carbonsäureamid- und Nitrilgruppen hydrolysiert, oder
(b) Amine II in an sich bekannter Weise mit einer Monohalogenessigsäure oder ihren $C_1$-$C_4$-Alkylestern oder einem Monohalogenacetonitril umsetzt und anschließend noch vorhandene Carbonsäureamid-, Carbonsäureester- und Nitrilgruppen hydrolysiert, oder
(c) Hydroxyaceton oder 1,3-Dihydroxyaceton mit Iminoverbindungen der allgemeinen Formel III

$(Y\text{-}CH_2)_2NH$ III

und mit Cyanwasserstoff oder Alkalimetallcyaniden in an sich bekannter Weise umsetzt und anschließend noch vorhandene Carbonsäureamid-, Carbonsäureester- und Nitrilgruppen hydrolysiert, oder
(d) für den Falle, daß Z eine Hydroxylgruppe bezeichnet, Nitrilotriacetonitril mit Formaldehyd in an sich bekannter Weise umsetzt und anschließend die Nitrilgruppen hydrolysiert.

3. Verfahren zum Komplexieren von Schwermetall- oder Erdalkalimetallionen, dadurch gekennzeichnet, daß man hierzu 2-Methyl- oder 2-Hydroxymethyl-serin-N,N-diessigsäure oder ihre Derivate I gemäß Anspruch 1 verwendet.

4. Verfahren zum Waschen und Reinigen, dadurch gekennzeichnet, daß man hierzu 2-Methyl- oder 2-Hydroxymethyl-serin-N,N-diessigsäure oder ihre Derivate I gemäß Anspruch 1 als Bleichmittelstabilisatoren oder Gerüststoffe verwendet.

5. Mittel zur Komplexierung von Schwermetall- oder Erdalkalimetallionen, enthaltend 0,01 bis 99 Gew.-%, bezogen auf die Gesamtmenge der Zubereitung, einer oder mehrerer Verbindungen der allgemeinen Formel I gemäß Anspruch 1.

6. Wasch- und Reinigungsmittel, enthaltend 0,01 bis 20 Gew.-%, bezogen auf die Gesamtmenge der Zubereitung, einer oder mehrerer Verbindungen der allgemeinen Formel I gemäß Anspruch 1.

## Claims

**1.** 2-Methyl- and 2-hydroxymethyl-serine-N,N-diacetic acids and their derivatives of the general formula I

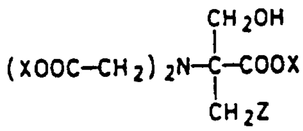

I

where X is hydrogen, alkali metals or ammonium which can be substituted by $C_1$-$C_4$-alkyl groups or $C_1$-$C_4$-hydroxyalkyl groups, and Z is hydrogen or a hydroxyl group.

**2.** A process for preparing 2-methyl- and 2-hydroxymethyl-serine-N,N-diacetic acids and their derivatives I as claimed in claim 1, which comprises
(a) reacting amines of the general formula II

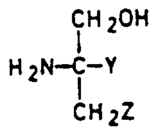

II

where Y is COOR, $CONH_2$ or CN, wherein R is hydrogen, an alkali metal, ammonium or a $C_1$-$C_4$-alkyl radical, with formaldehyde and hydrogen cyanide or alkali metal cyanides in a conventional manner and subsequently hydrolyzing carboxamide and nitrile groups still present, or
(b) reacting amines II in a conventional manner with a monohaloacetic acid or its $C_1$-$C_4$-alkyl esters or with a monohaloacetonitrile and subsequently hydrolyzing carboxamide, carboxylate and nitrile groups still present, or
(c) reacting hydroxyacetone or 1,3-dihydroxyacetone with imino compounds of the general formula III

$(Y\text{-}CH_2)_2NH$ III

and with hydrogen cyanide or alkali metal cyanides in a conventional manner and subsequently hydrolyzing carboxamide, carboxylate and nitrile groups still present, or
(d) when Z is hydroxyl, reacting nitrilotriacetonitrile with formaldehyde in a conventional manner and subsequently hydrolyzing the nitrile groups.

**3.** A method of complexing heavy metal or alkaline earth metal ions, which comprises using 2-methyl- or 2-hydroxymethyl-serine-N,N-diacetic acid or its derivatives I as claimed in claim 1.

**4.** A method of washing and cleaning, which comprises using 2-methyl- or 2-hydroxymethyl-serine-N,N-diacetic acid or its derivatives I as claimed in claim 1 as bleaching agent stabilizers or builders.

**5.** A composition for complexing heavy metal or alkaline earth metal ions comprising 0.01 to 99% by weight, based on the total amount of the preparation, of one or more compounds of the general formula I as claimed in claim 1.

**6.** A washing and cleaning composition comprising from 0.01 to 20% by weight, based on the total amount of the preparation, of one or more compounds of the general formula I as claimed in claim 1.

## Revendications

1. Acides 2-méthyl- et 2-hydroxyméthyl-sérine-N,N-diacétiques et leurs dérivés de formule générale I

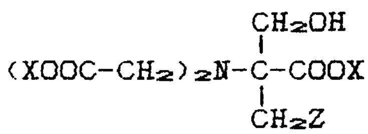

I

dans laquelle X est mis pour un atome d'hydrogène, des métaux alcalins ou un ion ammonium, qui peut être substitué par des groupes alkyle en $C_1$-$C_4$ ou hydroxyalkyle en $C_1$-$C_4$, et Z désigne un atome d'hydrogène ou un groupe hydroxyle.

2. Procédé de préparation d'acides 2-méthyl- et 2-hydroxyméthyl-sérine-N,N-diacétiques et de leurs dérivés I selon la revendication 1, caractérisé en ce que
(a) l'on fait réagir d'une manière connue en soi des amines de formule générale II

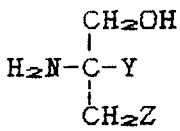

II

dans laquelle Y représente les groupes COOR, $CONH_2$ ou CN, R étant mis pour un atome d'hydrogène, un métal alcalin, un ion ammonium ou un reste alkyle en $C_1$-$C_4$, avec du formaldéhyde et de l'acide cyanhydrique ou des cyanures de métal alcalin, puis l'on hydrolyse les groupes amide d'acide carboxylique et nitrile encore présents, ou
(b) en ce que l'on fait réagir des amines II de manière connue en soi avec un acide monohalogénoacétique ou ses esters d'alkyle en $C_1$-$C_4$, ou un monohalogénoacétonitrile, puis on hydrolyse les groupes amide d'acide carboxylique, ester d'acide carboxylique et nitrile encore présents, ou
(c) on fait réagir l'hydroxyacétone ou la 1,3-dihydroxyacétone avec des composés imino de formule générale III

$(Y\text{-}CH_2)_2NH$ III

et avec de l'acide cyanhydrique ou des cyanures de métal alcalin, d'une manière connue en soi, puis on hydrolyse les groupes amide d'acide carboxylique, ester d'acide carboxylique et nitrile encore présents, ou
(d) dans le cas où Z représente un groupe hydroxyle, on fait réagir le nitrilotriacétonitrile avec du formaldéhyde d'une manière connue en soi, puis on hydrolyse les groupes nitrile.

3. Procédé de complexation d'ions de métal lourd ou de métal alcalino-terreux, caractérisé en ce que l'on utilise à cette fin des acides 2-méthyl- ou 2-hydroxyméhyl-sérine-N,N-diacétiques ou leurs dérivés I selon la revendijcation I.

4. Procédé de lavage et de purificaqtion, caractérisé en ce que l'on utilise à cette fin, comme stabilisants d'agents de blanchiment ou adjuvants de lavage, des acides 2-méthyl- ou 2-hydroxyméthyl-sérine-N,N-diacétiques ou leurs dérivés I selon la revendication 1.

5. Agent de complexation d'ions de métal lourd ou de métal alcalino-terreux, contenant 0,01 à 99% en poids, par rapport à la quantité totale de la préparation, d'un ou plusieurs composés de formule générale I selon la revendication 1.

6. Agent de lavage et de purification, contenant 0,01 à 20% en poids, par rapport à la quantité totale de la préparation, d'un ou plusieurs composés de formule générale I selon la revendication 1.